# FASCICULE DE BREVET EUROPEEN

(11) **EP 1 704 961 B1**
(45) Date de publication et mention de la délivrance du brevet: **29.10.2008**
(21) Numéro de dépôt: 05102315.8
(22) Date de dépôt: 22.03.2005
(51) Int. Cl.: B24B 1/00, B24B 11/00, B24B 15/00, A61F 2/44

(54) **Procédé de rectification de pièces concaves, notamment pour la fabrication de prothèses**
Verfahren zum Schleifen von konkaven Werkstücken, insbesondere von Prothesen
Method for grinding concave workpieces, in particular of prostheses

(43) Date de publication de la demande: 27.09.2006
(73) Titulaire: Voumard Machines Co. S.A., 2068 Hauterive (CH)
(72) Inventeur: Guenin, Maurice, 2300 La Chaux-de-Fonds (CH)
(74) Mandataire: P&TS Patents & Technology Surveys SA

(56) Documents cités:
- EP-A- 0 560 141
- FR-A- 2 151 191
- FR-A- 2 718 635
- GB-A- 152 606
- GB-A- 2 317 584
- US-A- 3 812 623
- US-A- 5 187 900
- US-A- 5 425 773
- PATENT ABSTRACTS OF JAPAN vol. 009, no. 025 (M-355), 2 février 1985 (1985-02-02) & JP 59 169756 A (HITACHI SEISAKUSHO KK), 25 septembre 1984 (1984-09-25)

## Description

### Domaine technique

La présente invention concerne un procédé pour rectifier des concavités, notamment pour la fabrication de prothèses cervicales ou d'autres types de prothèses.

### Etat de la technique

Les vertèbres cervicales et les disques cartilagineux assurant la liaison entre les vertèbres cervicales peuvent subir des tassements ou déformations provoqués par des chocs ou de mouvements répétitifs. II a déjà été proposé dans l'art antérieur de remplacer les vertèbres ou les disques altérés par des prothèses. La demande de brevet FR2718635 décrit une prothèse cervicale comprenant un plateau supérieur avec une surface concave et un plateau inférieur avec une surface convexe, destinée à pivoter dans la surface concave du plateau supérieur. Une prothèse similaire est aussi décrite dans US5562738.

L'état de surface des surfaces concaves et convexes des deux plateaux doit être parfaitement lisse afin de réduire les frottements, qui rendraient la prothèse sensible à l'usure et gèneraient les mouvements de flexion. Dans le cas de telles prothèses, il est avantageux de rectifier les surfaces convexes et concaves après ébauchage.

La présente invention concerne un procédé pour fabriquer de telles pièces, notamment un procédé pour rectifier la portion concave d'un des deux plateaux.

La rectification est effectuée sur une machine de rectification à l'aide d'un outil de rectification, par exemple une meule. La meule doit être munie d'une tige dont le diamètre doit être suffisant pour limiter l'amplitude des vibrations indésirables lors de la rectification et atteindre ainsi la précision requise. Il est cependant nécessaire de veiller à éviter les collisions entre l'outil, en particulier la tige de l'outil, et la pièce à usiner.

La surface concave de la prothèse supérieure a avantageusement la forme d'une calotte sphérique ou ellipsoïdale. La forme de l'outil de rectification ainsi que les mouvements relatifs de l'outil et de la pièce doivent être adapté pour rectifier une telle forme.

Les concavités des prothèses cervicales décrites dans les documents mentionnés ne sont pas rectifiées, en sorte que leur qualité est insuffisante. Des procédés de rectification à l'aide de déplacements d'axes complexes, faisant appel à des interpolations, pourraient être imaginés. La programmation de tels déplacements est cependant complexe. Un travail de programmation important doit être entrepris pour programmer la rectification de concavités différentes, par exemple pour fabriquer une gamme de prothèses pour remplacer différentes vertèbres d'individus de taille variable.

Un but de la présente invention est donc de proposer un procédé pour rectifier des concavités préalablement ébauchées dans une face d'une pièce, notamment d'une prothèse osseuse, qui soit simple à mettre en oeuvre et permette d'obtenir la qualité de surface requise. Un procédé connu de l'art antérieur pour rectifier des concavités est divulgué dans le document US-A-5 187 900.

Selon l'invention, ces objectifs sont atteints notamment au moyen d'un procédé de rectification de concavités allongées préalablement ébauchées dans une face d'une pièce, notamment d'une prothèse, ladite concavité ayant une section sensiblement en forme de portion de cercle dans un plan perpendiculaire à l'axe de rotation d'un outil, le procédé comprenant les étapes suivantes :
positionnement d'un outil de rectification dans une position de repos,
chargement de la pièce à rectifier,
déplacement relatif de l'outil de rectification et de la pièce à rectifier selon un axe parallèle audit axe de rotation de l'outil
déplacement relatif de l'outil de rectification et de la pièce selon un axe (X) perpendiculaire à l'axe de rotation de l'outil, de manière à amener l'outil en position de début de rectification,
rectification de la concavité en combinant des mouvements de rotation de l'outil de rectification et des mouvements de rotation de la pièce autour de deux axes parallèles entre eux.

La pièce à rectifier est chargée et serrée dans une pince porte-pièce rattachée au mandrin lui-même fixé sur la broche indexée de la poupée porte-pièce (axe C). La pièce à rectifier est placée sur le mandrin de manière à ce que le centre (axe) virtuel de la concavité à rectifier soit confondu avec l'axe de rotation de la pièce et de la poupée porte-pièce lors de la rectification.

Ce procédé a l'avantage de permettre la rectification de concavités de formes variables de manière simple et avec un minimum de déplacements complexes.

Des exemples de mise en oeuvre de l'invention sont indiqués dans la description illustrée par les figures annexées dans lesquelles :
La figure 1A illustre une vue en perspective d'une pièce dont la face inférieure comporte une concavité en forme de calotte d'ellipsoïde.
La figure 1B illustre une vue de dessous de la pièce de la figure 1A.
La figure 1C illustre une vue de côté, dans la direction de l'axe Y, de la pièce de la figure 1A.
La figure 1D illustre une vue de côté, dans la direction de l'axe Z, de la pièce de la figure 1A.
La figure 2 illustre une vue en coupe longitudinale de la partie abrasive d'une meule de rectification adaptée à la rectification de la pièce illustrée sur les figures 1A à 1D.
La figure 3A illustre une vue en perspective d'une pièce dont la face inférieure comporte une concavité en forme de calotte sphérique.
La figure 3B illustre une vue de dessous de la pièce de la figure 3A.
La figure 3C illustre une vue de côté, dans la direction de l'axe Y, de la pièce de la figure 3A.
La figure 3D illustre une vue de côté, dans la direction de l'axe Z, de la pièce de la figure 2A.
La figure 4 illustre une vue en coupe longitudinale de la partie abrasive d'une meule de rectification adaptée à la rectification de la pièce illustrée sur les figures 3A à 3D.
Les figures 5A à 5H montrent des vues de face (5A-5D) et de dessus (5E-5H) d'un dispositif pour la rectification d'une seule pièce par cycle, au cours de quatre étapes successives d'un cycle de rectification.
Les figures 6A à 6H montrent des vues de face (6A-6D) et de dessus (6E-6H) d'un dispositif pour la rectification simultanée de deux pièces par cycle, au cours de quatre étapes successives d'un cycle de rectification.
Les figures 7A à 7H montrent des vues de face (7A-7D) et de dessus (7E-7H) d'un dispositif pour l'ébauchage d'une pièce, au cours de quatre étapes successives d'un cycle d'ébauchage.
Les figures 8A à 8L montrent des vues de face (8A-8F) et de dessus (8G-8LH) d'un dispositif pour l'ébauchage simultané de deux pièces par cycles, au cours de six étapes successives d'un cycle d'ébauchage.
La figure 9 illustre en perspective, de manière schématique simplifiée, une première variante de machine de rectification apte à mettre en oeuvre le procédé de l'invention.
La figure 10 illustre en perspective, de manière schématique simplifiée, une deuxième variante de machine de rectification apte à mettre en oeuvre le procédé de l'invention.
La figure 11 illustre une vue en coupe selon le plan (X-Z) d'une pièce prête à être rectifiée.
La figure 12 illustre une vue en coupe selon le plan (X-Y) d'une pièce en cours de rectification.

### Exemple(s) de mode de réalisation de l'invention

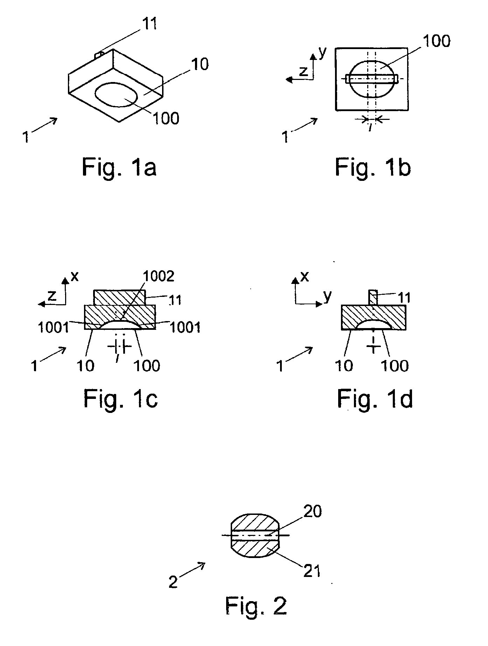

Un premier exemple de pièce à rectifier selon le procédé de l'invention est illustré sur les figures 1A à 1D. La pièce 1 illustrée correspond à un élément de prothèse cervicale ou interdiscale représentée de manière simplifiée. Elle est de préférence réalisée en métal, par exemple en acier chirurgical, en titane ou en céramique. Sa face supérieure est munie de moyens de fixation 11 pour la fixer sur l'organisme du patient, par exemple une patte destinée à être cimentée sur un os sain. La face inférieure 10 est munie d'une concavité 100, ou alvéole, dans laquelle une vertèbre cervicale ou une prothèse cervicale peut pivoter et se déplacer avec un minimum de frottement

La concavité des figures 1A à 1D a sensiblement une forme de cuvette sphérique allongée (« baignoire »), ou plus généralement de calotte d'ellipsoïde ; le bord de la calotte, illustré sur la figure 1B, est donc au moins approximativement en forme d'ellipse ou d'ovale. La section de l'ellipsoïde dans le plan X, Z, illustré sur la figure 1C, est au moins approximativement ovale ou elliptique. Le rebord 1001 a par exemple une forme de segment de cercle tandis que le fond 1002 est de préférence cylindrique. Dans le plan X, Y illustré sur la figure 1D, la calotte a en revanche une section sensiblement circulaire. Cette concavité permet ainsi à l'élément convexe de la portion de prothèse associée de se déplacer le long de l'axe Z et d'être mieux maintenu le long de l'axe Y.

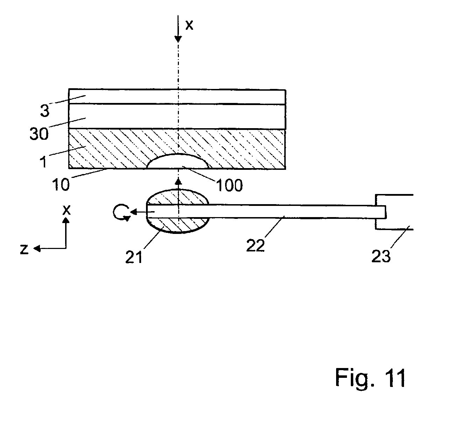

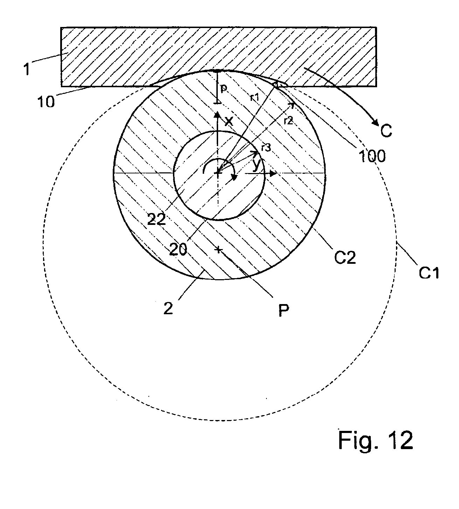

La figure 2 montre un exemple d'outil de rectification, ici une meule, appropriée pour rectifier la concavité 100 de la pièce illustrée sur les figures 1A à 1D. L'outil comporte une partie abrasive 21 en forme d'ellipsoïde correspondant à la forme de la concavité 100. La figure 12 est une vue en coupe dans le plan X, Y de l'outil 2 partiellement engagé dans la concavité 100. La section de l'outil dans ce plan est sphérique, mais son rayon r2 est inférieur au rayon de courbure r1 de la concavité dans le même plan. L'outil comporte en outre un trou 20 pour le monter sur une tige 22 permettant de charger l'outil sur une broche de rectification 23 (figure 11) de la machine à rectifier. Le diamètre de l'ouverture 20 et de la tige 22 est de préférence aussi grand que possible afin de maintenir fermement l'outil tout en évitant le risque de collision entre la tige 22 et la face à usiner 10.

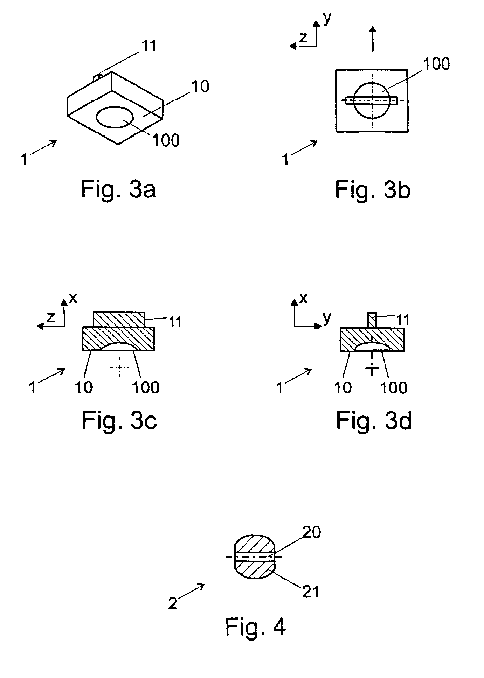

La concavité 100 illustrée sur les figures 1A à 1d peut aussi être rectifiée avec la meule à profil sphérique illustrée sur la figure 4 ; dans ce cas, un déplacement en z de la meule 2 relativement à la pièce 1 est nécessaire.

La variante de pièce illustrée sur les figures 3B à 3D est similaire à la variante des pièces 1 B à 1 D, mais la concavité 100 a une forme de calotte sphérique ; le bord de la concavité dans le plan Y-Z, illustré sur la figure 3B, est donc circulaire. Cette concavité peut être rectifiée avec la meule de la figure 4, dont la surface externe abrasive s'intègre dans une sphère.

Les formes des meules des figures 2 et 4 peuvent être obtenues par dressage avec un porte-diamant avec un déplacement angulaire contrôlé numériquement. La meule allongée de la figure 2 sera dressée en trois étapes continues (dressage de la première partie du rayon, puis déplacement en z de longueur I, puis dressage de la fin du rayon. La meule de la figure 4 peut être dressée en une seule étape. Le diamètre externe de la meule diminue après chaque dressage, mais le profil de la portion abrasive de la meule dans le plan x-z reste de préférence constant.

Dans une variante, les meules peuvent être dressées à l'aide d'une molette diamantée par interpolantion x et z, afin d'obtenir les formes respectives de meules de la figure 2 et 4.

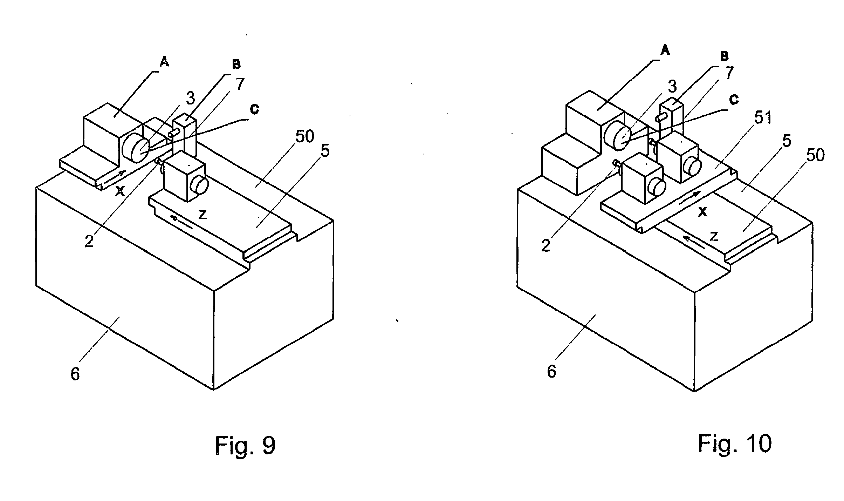

Un exemple de machine de rectification pouvant être programmée pour exécuter le procédé de l'invention est illustré de manière schématique sur la figure 9. La machine comporte un bâti 6 sur lequel est monté une table 5 permettant de déplacer une coulisse 50 selon un axe Z horizontal. Un outil de rectification 2 est monté à l'aide d'une broche de rectification non illustrée sur la coulisse 50, et peut tourner au moyen d'un moteur autour d'un axe parallèle à Z.

La pièce à rectifier est tenue dans la pince d'un mandrin sur la poupée 3 apte à tourner selon un axe C parallèle à l'axe horizontal Z. La poupée 3 peut en outre être déplacée par rapport au bâti 6 selon un axe X horizontal perpendiculaire à l'axe Z. La machine peut en outre comporter des éléments de dressage 7.

Une variante de machine de rectification plus complexe est illustrée sur la figure 10. Cette machine comporte, en plus des éléments déjà présents sur la machine de la figure 9, une deuxième coulisse 51 sur la table 5 afin de déplacer les outils selon l'axe X, au lieu de déplacer la poupée 3. Cette machine comporte en outre deux broches de rectification parallèles et entraînées indépendamment l'une de l'autre.

D'autres configurations de machines de rectification permettant au moins de déplacer la pièce et l'outil relativement l'un à l'autre selon les axes X, Z et C peuvent être imaginés dans le cadre de l'invention.

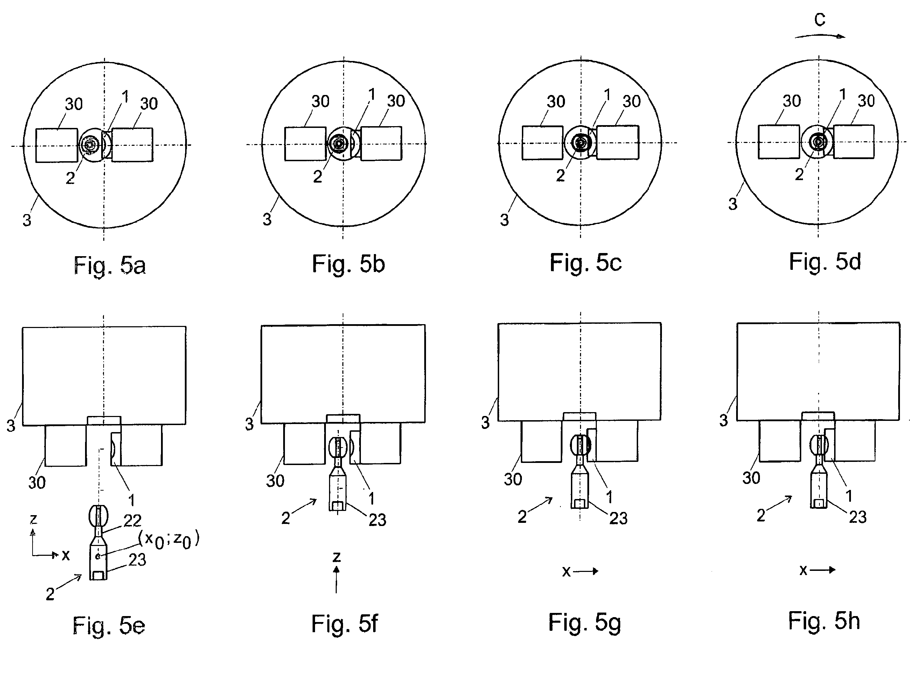

Les figures 5E à 5H montrent des vues de face (5A-5D) et de dessus (5E-5H) d'un dispositif pour la rectification d'une seule pièce par cycles, au cours de quatre étapes successives d'un cycle. Dans cette variante, les déplacements linéaires de l'outil de rectification 2 par rapport à la pièce 1 sont effectués par des mouvements de la table 5 portant l'outil 2 selon les axes horizontaux X et Z ; les mouvements en X et/ou en Z pourraient cependant aussi être obtenus par déplacement de la pièce. La pièce 1 peut en outre tourner autour d'un axe C horizontal. L'axe C est parallèle mais distinct de l'axe de rotation de l'outil; les déplacements en X permettent d'éloigner ou de rapprocher ces deux axes.

Dans la position illustrée sur les figures 5A et 5E, la pièce à rectifier, préalablement ébauchée, a déjà été chargée dans la pince du mandrin 30. L'outil 2 est dans une position de repos X0, Z0 sans risque de collision avec la pièce 1. Le mandrin est indexé dans une position prédéfinie selon l'axe C. Cette position permet l'engagement de l'outil, par exemple une meule, à l'intérieur de la concavité en évitant tout risque de collision.

Sur les figures 5B et 5F, l'outil est déplacé selon l'axe Z vers une position en regard et à distance de la concavité 100. Le mandrin est toujours indexé. Cette position est illustrée agrandie sur la figure 11.

Sur les figures 5C et 5G, l'outil est déplacé selon l'axe X uniquement en position de début de rectification. La partie abrasive de l'outil est alors partiellement engagée dans la concavité 100, dans la position illustrée sur la figure 12.

Le mandrin 30 qui porte la pièce 1 est mis en rotation selon l'axe C dans la position illustrée sur les figures 5D et 5H. La rectification commence, et combine des rotations de la pièce selon l'axe C avec des déplacements de l'outil selon l'axe X (approche rapide puis fonçage), afin d'effectuer la finition puis la superfinition de la concavité.

Au terme de la rectification, l'outil est dégagé dans la position de début de rectification, le mandrin est arrêté et indexé, puis l'outil est complètement dégagé en position X0 et finalement retiré en position Z0. La pièce rectifiée peut ensuite être déchargée pour recommencer un cycle avec une nouvelle pièce.

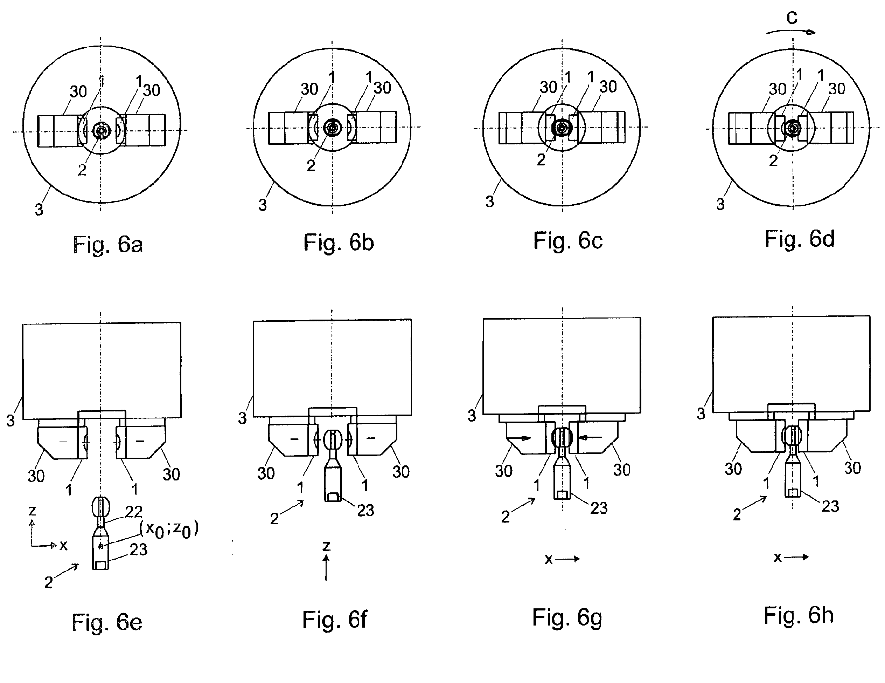

Les figures 6A à 6H montrent des vues de face (6A-6D) et de dessus (6E-6H) d'une machine dont le mandrin 30 comporte deux pinces non représentées placées chacune sur une navette pour la rectification simultanée de deux pièces 1 en regard l'une de l'autre au cours du même cycle. Les deux navettes peuvent être en position ouvertes, vers l'extérieur du mandrin, pour le chargement, ou fermée vers le centre du mandrin lors de la rectification. En position fermée, les positions des navettes sont telles que les centres virtuels des profils rectifiés se confondent avec l'axe du mandrin. L'entrée et la sortie de l'outil de rectification dans les concavités peut se faire uniquement lorsque les navettes sont en position ouverte.

Sur les figures 6A et 6E, les deux pièces ont été chargées dans leurs pinces respectives, et l'outil 2 est en retrait en position X0, Z0. X0 correspond au centre de la poupée porte-pièce 3. Le mandrin est indexé.

Sur les figures 6B et 6F, le mandrin est toujours immobile et indexé, l'outil est avancé selon l'axe Z en regard des deux concavités, centré par rapport à la poupée. Les mors se ferment ensuite dans la position illustrée sur les figures 6C et 6G, ce qui revient à déplacer les deux pièces 1 dans la même direction X et dans un sens opposé, de manière à les rapprocher de l'outil 2. La partie abrasive de l'outil est alors partiellement engagée dans les deux concavités 100 des deux pièces, en position de début de rectification.

Le mandrin est ensuite mis en rotation selon l'axe C, ce qui permet de commencer la rectification illustrée sur les figures 6D et 6H. La rectification combine des approches rapides selon l'axe X, par déplacement de l'outil 2, et des fonçages en X, de manière à effectuer une finition puis une superfinition de la pièce.

Au terme de la rectification, l'outil retourne en position X0 au centre de la broche, le mandrin est arrêté et indexé, puis les mors sont ouverts pour permettre le dégagement de l'outil selon l'axe Z jusqu'à la position initiale Z0. Les pièces rectifiées peuvent ensuite être déchargées afin de recommencer un cycle avec de nouvelles pièces.

Selon la géomètrie des pièces à usiner, et en effectuant éventuellement des déplacements d'axes supplémentaires, il est aussi possible, dans le cadre de l'invention, d'usiner simultanément plus de deux pièces au cours d'un cycle et en employant le même outil.

Comme on le voit en particulier sur la figure 12, l'outil de rectification 2 tourne autour de l'axe Z et la pièce tourne autour du deuxième axe C distinct de Z. L'axe C passe par le centre virtuel P du profil, c'est-à-dire par le centre du cercle C1 définissant la concavité 100 dans le plan de la page. Par ailleurs, le rayon r2 de la section circulaire de l'outil 2 dans le plan X, Y est nettement inférieur au rayon r1 de la concavité 100. Dans ce contexte, nettement inférieur signifie que la différence n'est pas due au hasard ni au jeu nécessaire, mais que la différence est intentionnelle, et permet une rotation relative de l'outil et de la pièce selon l'axe C.

La forme et la dimension de la concavité dépend donc à la fois de la forme de la meule et de la trajectoire parcourue par la pièce autour de la meule.

Afin d'éviter les collisions, il est important que la différence entre le rayon maximal r2 de la portion abrasive 21 et le rayon r3 de la tige 22 soit plus importante que la profondeur maximale p de la concavité 100. On choisira cependant de préférence une tige d'un diamètre maximal respectant cette condition, en tenant compte d'une marge de sécurité pour éviter les collisions dues aux tolérances de fabrication et aux déformations de l'outil et/ou de la pièce lors de la rectification.

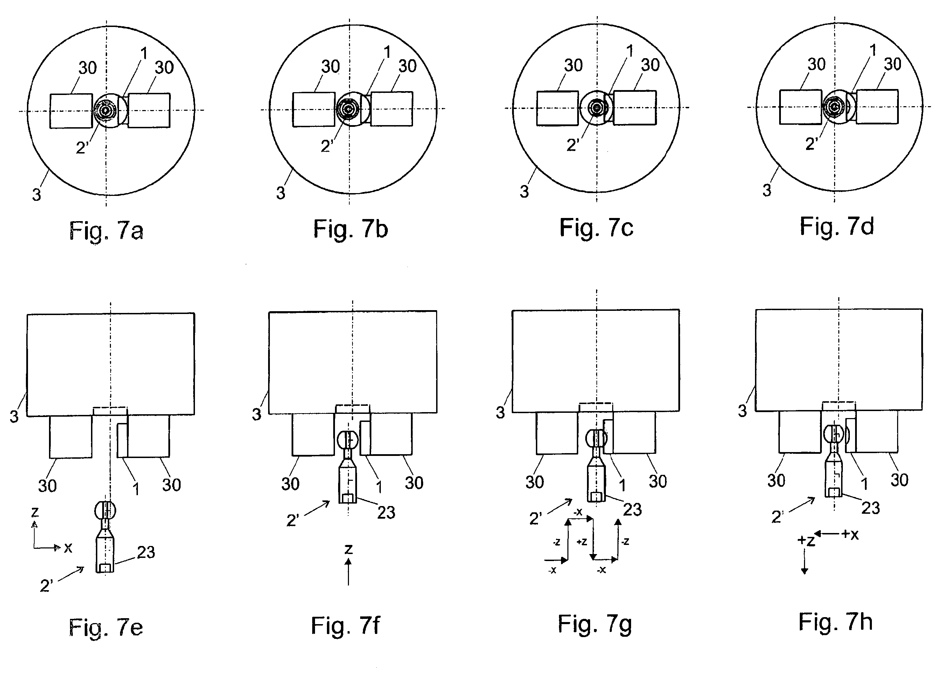

Les figures 7A à 7H illustrent les opérations effectuées pour ébaucher la concavité 100 dans une pièce préalablement usinée sur une machine-outil conventionnelle. L'ébauchage peut être effectué sur la machine de rectification décrite plus haut, en remplaçant l'outil de rectification 2 par un outil d'ébauchage 2', ou sur une autre machine effectuant uniquement l'ébauchage et permettant des déplacements selon les mêmes axes. Dans une variante préférentielle, l'ébauchage et la rectification sont effectués tous deux sur une seule machine comportant deux broches d'usinage, comme illustré sur la figure 10, une des broches étant munie d'un outil d'ébauchage 2', par exemple une fraise ou une meule galvanique sphérique, l'autre broche d'un outil de rectification 2, par exemple d'une meule de rectification.

Sur les figures 7A et 7E, la pièce 1 est chargée, le mandrin est indexé, l'outil 2' est en position de repos X0, Z0. L'outil est ensuite déplacé selon l'axe Z, dans la position illustrée sur les figures 7B et 7F, en regard de la concavité à usiner.

L'usinage de la concavité 100 est effectué sur les figures 7C et 7G par une combinaison d'avances en X et en Z, le mandrin 3 restant toujours indexé et n'étant pas mis en rotation. Après ébauchage, l'outil 2' est dégagé par un premier mouvement de recul selon l'axe X, puis un deuxième mouvement de recul selon l'axe Z(Figures 7D-7H), de manière à retourner à la position initiale X0 Z0 permettant le déchargement de la pièce (si nécessaire), ou son déplacement selon l'axe X en regard de l'outil de rectification sur la même machine.

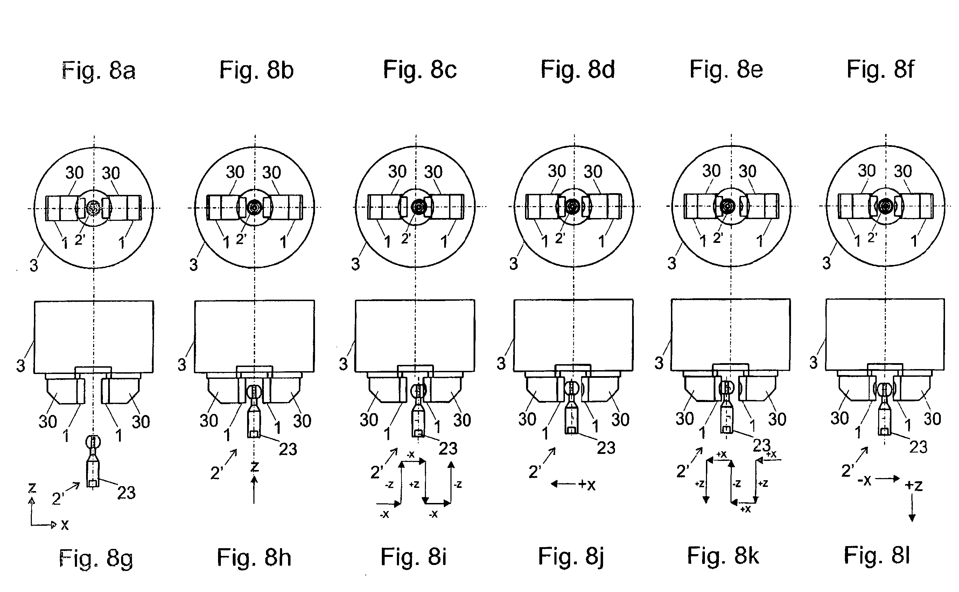

Les figures 8A à 8L illustrent les opérations effectuées pour ébaucher la concavité 100, à l'aide d'une machine permettant d'ébaucher plusieurs pièces lors du même cycle.

Comme pour la variante de la figure 7, l'ébauchage est avantageusement, mais pas nécessairement, effectué sur la machine effectuant également la rectification et munie à la fois d'une broche d'ébauchage et d'une broche de rectification.

Sur les figures 8A et 8G, la pièce 1 est chargée, le mandrin est indexé, l'outil 2' est en position de repos X0, Z0. L'outil est ensuite déplacé selon l'axe Z, dans la position illustrée sur les figures 8B et 8H, en regard des deux concavités à usiner dans les deux pièces en regard 1.

L'usinage de la concavité 100 dans la première pièce (à droite de l'outil sur la figure) est effectué sur les figures 8C et 8I en déplaçant l'outil 2' vers cette première pièce et/ou en fermant le mors maintenant cette première pièce. L'ébauchage est effectué par une combinaison d'avances en X et en Z, le mandrin 3 restant toujours indexé et n'étant pas mis en rotation.

Après ébauchage de la première pièce, l'outil d'ébauchage 2' est ensuite dégagé en position X0 (figures 8D et 8J), puis rapproché de la deuxième pièce (à sa gauche sur les figures) par un déplacement de l'outil selon l'axe X et/ou en fermant le mors maintenant la deuxième pièce. L'usinage de la deuxième concavité est ensuite effectué en combinant des déplacements en X et en Z, le mandrin étant immobile, comme illustré sur les figures 8E et 8K.

Après ébauchage des concavités sur les deux pièces, l'outil 2' est dégagé par un premier mouvement de recul selon l'axe X, puis un deuxième mouvement de recul selon l'axe Z, de manière à retourner à la position initiale X0 Z0 permettant le déchargement de la pièce (si nécessaire), ou son déplacement selon l'axe X en regard de l'outil de rectification sur la même machine (Figures 8F-8L).

La machine d'ébauchage et de rectification peut aussi être utilisée pour effectuer d'autres opérations d'usinage sur la même pièce 1, par exemple pour ébaucher et ou rectifier d'autres portions concaves ou convexes sur la face 10 de la pièce, pour rectifier la face 10, etc. Ces opérations complémentaires peuvent être effectuées avec les mêmes outils, ou avec des outils différents de ceux employés dans les opérations décrites plus haut.

### Liste de pièces

- 1: Pièce à rectifier
- 10: Face de la pièce comportant une concavité
- 100: Concavité
- 1001: Bord arrondi de la concavité
- 1002: Fond cylindrique de la concavité
- 11: Moyens de fixation sur l'organisme
- 2: Outil de rectification (meule)
- 20: Trou de fixation de la tige
- 21: Partie abrasive
- 22: Tige
- 23: Broche de rectification
- 3: Poupée porte-pièce
- 30: Mandrin porte-pièce
- 5: Table
- 50: Coulisse Z
- 51: Coulisse X
- 6: Bâti
- 7: Moyens de dressage
- r1: Rayon de courbure de la concavité dans le plan X, Y
- r2: Rayon de courbure de l'outil de rectification dans le plan X, Y

## Revendications

1. Procédé de rectification de concavités (100) allongées préalablement ébauchées dans une face (10) d'une pièce (1), notamment d'une prothèse, ladite concavité ayant une section sensiblement en forme de portion de cercle dans un plan perpendiculaire à l'axe de rotation d'un outil, le procédé comprenant les étapes suivantes :
positionnement d'un outil de rectification (2) dans une position de repos (X0, Z0),
chargement de la pièce (1) à rectifier,
déplacement relatif de l'outil de rectification (2) et de la pièce à rectifier (1) selon un axe (Z) parallèle audit axe de rotation de l'outil,
déplacement relatif de l'outil de rectification (2) et de la pièce à rectifier (1) selon un axe (X) perpendiculaire à l'axe de rotation de l'outil, de manière à amener l'outil en position de début de rectification,
rectification de la concavité (100) en combinant des mouvements de rotation de l'outil de rectification et des mouvements de rotation de la pièce (1) autour de deux axes (Z, C) parallèles entre eux.

2. Le procédé de la revendication 1, dans lequel ledit axe de rotation est sensiblement parallèle à ladite face (10).

3. Le procédé de la revendication 1, dans lequel ledit outil est orienté parallèlement à la direction d'élongation maximale de ladite concavité.

4. Le procédé de la revendication 1, dans lequel lesdites concavités ont sensiblement une forme de callote d'ellipsoïde.

5. Le procédé de la revendication 1, dans lequel l'outil de rectification (2) tourne autour d'un premier axe (Z) et la pièce tourne autour d'un deuxième axe (C) distinct dudit premier axe.

6. Le procédé de la revendication 5, dans lequel le deuxième axe (C) est plus éloigné de la portion usinée que le premier axe (Z).

7. Le procédé de la revendication 1, dans lequel la concavité (100) est rectifiée en combinant des mouvements de rotation de l'outil de rectification et des mouvements de rotation de la pièce autour de deux axes (Z, C) parallèles entre eux, et des mouvements de déplacement relatif de l'outil (2) et de la pièce à rectifier (1) selon ledit axe (X) perpendiculaire à l'axe de rotation de l'outil.

8. Le procédé de la revendication 4, ladite pièce (1) étant rectifiée au moyen d'un outil (2) comportant une tige (22) et une portion abrasive (21) en bout de tige, la différence entre le rayon maximal (r2) de ladite portion abrasive (21) et le rayon (r3) de ladite tige (22) étant plus importante que la profondeur maximale (p) de ladite concavité (100).

9. Le procédé de la revendication 1, ladite concavité (100) ayant sensiblement la forme d'une calotte d'ellipsoïde ayant une section approximativement ovale ou elliptique dans un plan (X, Z) parallèle à l'axe (Z) de rotation de l'outil.

10. Le procédé de la revendications 9, ladite portion abrasive (22) de l'outil ayant sensiblement la forme d'une ellipsoïde ayant une section approximativement ovale ou elliptique dans un plan (X, Z) parallèle à l'axe (Z) de rotation de l'outil et une section sensiblement circulaire dans un plan (X, Y) perpendiculaire à l'axe (Z) de rotation de l'outil (2),
le rayon (r2) de ladite section en forme de portion de cercle de l'outil étant nettement inférieur au rayon (r1) de ladite section en forme de portion de cercle de la concavité (100).

11. Le procédé de la revendication 1, dans lequel l'axe de rotation (C) de la pièce lors de la rectification traverse le centre virtuel (P) de la concavité (100) à rectifier.

12. Le procédé de la revendication 1, dans lequel deux pièces (1) en regard l'une de l'autre sont rectifiées au cours du même cycle par le même outil (2).

13. Le procédé de la revendication 12, dans lequel ladite étape de déplacement relatif de l'outil de rectification et de la pièce selon un axe (X) perpendiculaire à l'axe de rotation de l'outil est effectué en déplaçant simultanément les deux dites pièces (1) dans des directions identiques selon des sens opposés, de manière à les rapprocher l'une de l'autres et de l'outil (2), puis en déplaçant l'outil vers l'une des deux pièces.

14. Le procédé de la revendication 1, comportant une étape préliminaire d'usinage de ladite pièce puis d'ébauchage de ladite concavité (100) dans ladite pièce (1).

15. Le procédé de la revendication 1, comportant une étape préliminaire d'ébauchage de ladite concavité (100) dans ladite pièce (1), ledit ébauchage étant effectué par une combinaison de déplacements selon un axe (Z) parallèle à l'axe de rotation de l'outil d'ébauchage (2') et de déplacements selon un axe (X) perpendiculaire à l'axe de rotation de l'outil d'ébauchage (2'), la pièce (1) étant immobile lors de l'ébauchage.

16. Le procédé de la revendication 14, ledit ébauchage et ladite rectification étant effectuées sur une même machine munie de deux broches d'usinage.

17. Le procédé de la revendication 14, dans lequel deux pièces (1) en regard l'une de l'autre sont ébauchées au cours du même cycle par le même outil (2).

## Claims

1. Method for grinding elongate concavities (100) which have previously been rough-machined into a surface (10) of a workpiece (1), in particular of a prosthesis, said concavity having a section essentially in the form of a portion of a circle in a plane perpendicular to the rotational axis of a tool, the method comprising the following steps:
positioning a grinding tool (2) in a home position (X0, Z0),
loading the workpiece (1) to be ground,
moving the grinding tool (2) and the workpiece (1) to be ground in relation to one another along an axis (Z) parallel to said rotational axis of the tool,
moving the grinding tool (2) and the workpiece (1) to be ground in relation to one another along an axis (X) perpendicular to the rotational axis of the tool so as to convey the tool to a position at which grinding starts, and
grinding the concavity (100) by combining rotary movements of the grinding tool with rotary movements of the workpiece (1) about the two mutually parallel axes (Z, C).

2. Method according to Claim 1, in which said rotational axis is essentially parallel to said surface (10).

3. Method according to Claim 1, in which said tool is oriented parallel to the line of maximum extent of said concavity.

4. Method according to Claim 1, in which said concavities are essentially in the form of an ellipsoidal cap.

5. Method according to Claim 1, in which the grinding tool (2) rotates about a first axis (Z), and the workpiece rotates about a second axis (C) different from said first axis.

6. Method according to Claim 5, in which the second axis (C) is further away from the machined portion than the first axis (Z).

7. Method according to Claim 1, in which the concavity (100) is ground by combining rotary movements of the grinding tool with rotary movements of the workpiece about two mutually parallel axes (Z, C), and movements of the tool (2) and the workpiece (1) to be ground in relation to one another along said axis (X) perpendicular to the rotational axis of the tool.

8. Method according to Claim 4, wherein said workpiece (1) is ground by a tool (2) comprising a stem (22) and an abrasive portion (21) at the end of the stem, the difference between the maximum radius (r2) of said abrasive portion (21) and the radius (r3) of said stem (22) being greater than the maximum depth (p) of said concavity (100).

9. Method according to Claim 1, wherein said concavity (100) is essentially in the form of an ellipsoidal cap having an approximately oval or elliptical section in a plane (X, Z) parallel to the rotational axis (Z) of the tool.

10. Method according to Claim 9, wherein said abrasive portion (22) of the tool is essentially in the form of an ellipsoid having an approximately oval or elliptical section in a plane (X, Z) parallel to the rotational axis (Z) of the tool and an essentially circular section in a plane (X, Y) perpendicular to the rotational axis (Z) of the tool (2),
the radius (r2) of said section in the form of a portion of a circle of the tool being much less than the radius (r1) of said section in the form of a portion of a circle of the concavity (100).

11. Method according to Claim 1, in which during grinding the rotational axis (C) of the workpiece passes through the virtual centre (P) of the concavity (100) to be ground.

12. Method according to Claim 1, in which two workpieces (1) facing one another are ground during a single cycle by the same tool (2).

13. Method according to Claim 12, in which said step of moving the grinding tool and the workpiece in relation to one another along an axis (X) perpendicular to the rotational axis of the tool is carried out by simultaneously moving said two workpieces (1) along parallel lines towards but in opposite directions so as to bring them closer together and to the tool (2), and then by moving the tool towards one of the two workpieces.

14. Method according to Claim 1, which comprises a preliminary step of machining said workpiece and then rough-machining said concavity (100) into said workpiece (1).

15. Method according to Claim 1, which comprises a preliminary step of rough-machining said concavity (100) into said workpiece (1), said rough-machining being carried out by a combination of movements along an axis (Z) parallel to the rotational axis of the rough-machining tool (2') and movements along an axis (X) perpendicular to the rotational axis of the rough-machining tool (2'), the workpiece (1) being stationary during the rough-machining.

16. Method according to Claim 14, wherein said rough-machining and said grinding are carried out on the same machine which is provided with two machining spindles.

17. Method according to Claim 14, in which two workpieces (1) facing one another are rough-machined during a single cycle by the same tool (2).

## Patentansprüche

1. Verfahren für das Schleifen von länglichen Wölbungen (100), welche zuerst in eine Oberfläche (10) eines Werkstücks (1), insbesondere von einer Prothese, grobbearbeitet worden sind, wobei besagte Wölbung einen Abschnitt im Wesentlichen in Form eines Teilkreises in einer Ebene senkrecht zu der Rotationsachse eines Werkzeugs hat, das Verfahren umfasst die folgenden Schritte:
Positionieren eines Schleifwerkzeugs (2) in einer Heimatposition (X0, Z0),
Laden des zu schleifenden Werkstücks (1),
Bewegen des Schleifwerkzeugs (2) und des zu schleifenden Werkstücks (1) in Beziehung zueinander entlang einer Achse (Z) parallel zu besagter Rotationsachse von besagtem Werkzeug,
Bewegen des Schleifwerkzeugs (2) und des zu schleifenden Werkstücks (1) in Beziehung zueinander entlang einer Achse (X) senkrecht zu der Rotationsachse des Werkzeugs, um so das Werkzeug zu einer Position zu führen, an welcher das Schleifen beginnt, und
Schleifen der Wölbung (100) durch eine Kombination von Rotationsbewegungen des Schleifwerkzeugs mit Rotationsbewegungen des Werkstücks (1) um die zwei gegenseitig parallelen Achsen (Z, C).

2. Verfahren gemäss Anspruch 1, in welchem besagte Rotationsachse im Wesentlichen parallel zu besagter Oberfläche (10) ist.

3. Verfahren gemäss Anspruch 1, in welchem besagtes Werkzeug parallel zu der Linie von maximaler Ausdehnung von besagter Wölbung ist.

4. Verfahren gemäss Anspruch 1, in welchem besagte Wölbungen im Wesentlichen in der Form einer ellipsoiden Kappe sind.

5. Verfahren gemäss Anspruch 1, in welchem das Schleifwerkzeug (2) um eine erste Achse (Z) rotiert, und das das Werkstück um eine zweite Achse (C) rotiert, die von besagter ersten Achse verschieden ist.

6. Verfahren gemäss Anspruch 5, in welchem die zweite Achse (C) von dem bearbeiteten Teil weiter als die erste Achse (Z) weg ist.

7. Verfahren gemäss Anspruch 1, in welchem die Wölbung (100) durch rotatorische Bewegungen des Schleifwerkzeugs mit rotatorischen Bewegungen des Werkstücks um zwei gegenseitig parallele Achsen (Z, C), und die Bewegungen des Werkzeugs (2) und des zu schleifenden Werkstücks (1) in Beziehung zueinander entlang besagter Achse (X) senkrecht zu der Rotationsachse des Werkzeugs geschliffen wird.

8. Verfahren gemäss Anspruch 4, wobei besagtes Werkstück (1) durch ein Werkzeug (2) geschliffen wird, welches einen Stamm (22) und ein abrasives Teil (21) an dem Ende des Stammes umfasst, wobei die Differenz zwischen dem maximalen Radius (r2) des abrasiven Teils (21) und dem Radius (r3) von besagtem Stamm (22) grösser ist, als die maximale Tiefe (p) von besagter Wölbung (100).

9. Verfahren gemäss Anspruch 1, wobei besagte Wölbung (100) im Wesentlichen in der Form einer ellipsoiden Kappe mit einem annähernd ovalen oder ellipsoiden Abschnitt in einer Ebene (X, Z) parallel zu der Rotationsachse (Z) des Werkzeugs ist.

10. Verfahren gemäss Anspruch 9, wobei besagter abrasiver Teil (22) des Werkzeugs im Wesentlichen in der Form eines Ellipsoids mit einem ovalen oder elliptischen Abschnitt in einer Ebene (X, Z) parallel zu der Rotationsachse (Z) des Werkzeugs ist und einen im Wesentlichen runden Abschnitt in einer Ebene (X, Y) senkrecht zu der Rotationsachse (Z) des Werkzeugs (2),
wobei der Radius (r2) von besagtem Abschnitt in Form eines Teilkreises des Werkzeugs sehr viel kleiner ist als der Radius (r1) von besagtem Abschnitt in der Form eines Teilkreises der Wölbung (100).

11. Verfahren gemäss Anspruch 1, in welchem während des Schleifens die Rotationsachse (C) des Werkstücks durch das virtuelle Zentrum (P) der zu schleifenden Wölbung (100) schleift.

12. Verfahren gemäss Anspruch 1, in welchem zwei gegenüberliegende Werkstücke (1) während eines einzigen Zyklus' durch dasselbe Werkzeug (2) geschliffen werden.

13. Verfahren gemäss Anspruch 12, in welchem besagter Schritt von Bewegen des Schleifwerkzeugs und des Werkstücks in Beziehung zueinander entlang einer Achse (X) senkrecht zu der Rotationsachse des Werkzeugs durch gleichzeitiges Bewegen besagter Werkstücke (1) entlang paralleler Linien in gleicher Richtung, die entgegengesetzt sind, durchgeführt wird, um diese näher zueinander und zu dem Werkstück (2) zu bringen und dann durch Bewegen des Werkzeugs in Richtung von einem der beidem Werkstücke.

14. Verfahren gemäss Anspruch 1, welches einen vorläufigen Schritt von Bearbeiten des besagten Werkstücks und dann Grobbearbeiten besagter Wölbung (100) in besagtes Werkstück (1) umfasst.

15. Verfahren gemäss Anspruch 1, welches einen ersten Schritt von Grobbearbeitung von besagter Wölbung (100) in besagtes Werkstück (1) umfasst, wobei besagte Grobbearbeitung durch eine Kombination von Bewegungen entlang einer Achse (Z) durchgeführt wird, die parallel zu der Rotationsachse des grobbearbeitenden Werkzeugs (2') ist, und Bewegungen entlang einer Achse (X), die senkrecht zu der Rotationsachse des grobbearbeitenden Werkzeugs (2') ist, wobei das Werkstück (1) während der Grobbearbeitung stationär ist.

16. Verfahren gemäss Anspruch 14, wobei besagte Grobbearbeitung und besagtes Schleifen auf derselben Maschine ausgeführt werden, welche mit zwei Bearbeitungsspindeln ausgestattet ist.

17. Verfahren gemäss Anspruch 14, in welchem zwei gegenüberliegende Werkstücke (1) während eines einzigen Zyklus' durch dasselbe Werkzeug (2) grob bearbeitet werden.
